# EUROPEAN PATENT APPLICATION

(11) **EP 1 306 385 A1**
(43) Date of publication of application: **02.05.2003**
(21) Application number: 01125709.4
(22) Date of filing: 27.10.2001
(51) Int. Cl.: C07K 14/435, G01N 33/53

(54) **Aequorin as a reporter gene in yeast**

(71) Applicant: Aventis Pharma Deutschland GmbH, 65929 Frankfurt am Main (DE)
(72) Inventor: Fraissignes, Pauline, 81369 München (DE); Guédin, Denis, Dr., 83440 Montauroux (FR)

(57) **Abstract**

The present invention relates to a modified yeast cell containing Aequorin as reporter gene for detection of signaling pathway activity and methods of using such modified yeast cells.

## Description

The present invention relates to a modified yeast cell containing Aequorin as reporter gene for detection of signaling pathway activity and methods of using such modified yeast cells.

Aequorin is a photoprotein isolated from luminescent jellyfish *Aequoria victoria.*

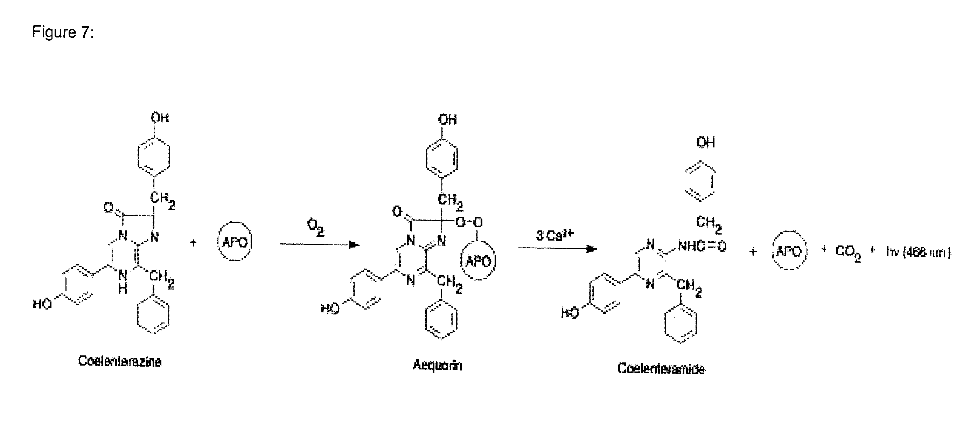

Apoaequorin is a protoprotein which, upon binding to coelenterazine, can emit photons in the presence Ca²⁺.
The Aequorin complex comprises a 22,514 MW Apoaequorin protein, molecular oxygen and the luminophore coelenterazine (Inouye *et al*., 1989;Johnson and Shimomura, 1978;Shimomura and Johnson, 1978). When three Ca²⁺ ions bind to this complex, coelenterazine is oxidized to coelenteramide, with a concomitant release of carbon dioxide and blue light (emission maximum ∼466 nm) (Figure 7).

Because of its Ca²⁺-dependent luminescence, the Aequorin complex has been extensively used as an intracellular Ca²⁺ indicator.

Aequorin reportedly does not disrupt cell functions or embryo development (Miller *et al*., 1994).

This photoprotein can be easily expressed in mammalian cells. It is utilized to monitor the cytosolic-free calcium concentration (Thomas and Delaville, 1991) (Sheu *et al*., 1993) (Stables *et al*., 2000).

Aequorin can also be easily targeted to specific organelles such as mitochondria (Brini *et al*., 1999) (Rizzuto *et al.,* 1992) to monitor different aspects of calcium homeostasis.

The pharmaceutical industry takes widely advantage of the different properties of Aequorin, and particularly in High Throughput Screens (Detheux, 2000). The activation of a receptor coupled to the phospholipase C transduction pathway can be easily detected in presence of the photoprotein Aequorin, because of an instantaneous release of calcium from the endoplasmic reticulum.
WO0002045, Detheux et al. (EUROSCREEN S.A.) describes a high-throughput screening diagnostic and/or dosage method of an agonist and/or an antagonist for a calcium-coupled receptor (in mammalian cells) where Aequorin is used as marker for intracellular calcium changes upon receptor stimulation.

It has been previously shown that Aequorin can be functionally expressed in yeast and detected.

Nakajima-Shimada et al. (Nakajima-Shimada *et al*., 1991b) describe the monitoring of intracellular calcium in *Saccharomyces cerevisiae* with an Apoaequorin cDNA expression system. Here, Aequorin was again used as a marker of intracellular calcium upon stress or glucose variations in the medium.

In contrast to mammalian signal transduction, there is no comparable Ca²⁺ release from the endoplasmic reticulum upon G protein-coupled receptor (GPCR) activation in yeast cells. The addition of α-factor to a cell (i.e. stimulation of the GPCR Ste2), from a basal level of approximately 100 nM, raises [Ca2+]i to a few hundred nanomolar in the cells, simultaneous with the induction of Ca2+ influx. When the cells are incubated with α- factor in a Ca2(+)-deficient medium, Ca2+ influx is greatly reduced, and the rise in [Ca2+]i is not detected (lida *et al*., 1990). This slide variation does not interfere with pathway activity detection in our experiments.

A limited number of reporter genes can be used in the yeast *Saccharomyces cerevisiae* and are not always appropriate. For screening purposes, the reporter product must be easy to detect and, for that reason, convenient reporter assays is required. The most commonly utilized reporter gene is LacZ which encodes the very big and stable enzyme β-Galactosidase.

Aequorin is a photoprotein detected like β-Galactosidase in a chemiluminescence assay.
As Aequorin (22514 MW) is five times smaller than β-Galactosidase (116351 MW), it can accumulate in a bigger amount to give a higher sensitivity to the assay and report better up- and down-regulations of the pathway (activating or inhibiting effect of the tested compound).

Additionally, bacterial contamination sometimes occurs in the yeast assay and most of the contaminants physiologically express a β-Galactosidase. The contaminated cultures would give a very strong signal in presence of the β-Galactosidase substrate. The use of Aequorin eliminates the risk of false positives due to contamination.

In summary, Aequorin is more suitable than β-Galactosidase.
Aequorin can be expressed functionally by the yeast *S. cerevisiae* (Nakajima-Shimada *et al*., 1991a).
Aequorin is easily detected in a luminescent assay, in present of coelenterazin and Ca2+.

So far Aequorin has not been utilized as a pathway activity reporter gene (as described for LacZ and HIS3, with mammalian G protein coupled receptor coupled to the yeast pheromone pathway (King *et al.,* 1990;Hadcock and Pausch, 1999).

The control of yeast mating signal transduction by a mammalian receptor (β₂-adrenergic) was first described in 1990 (King *et al.,* 1990).
US 5,876,951A claims yeast cells engineered to produce pheromone system protein surrogates and uses therefor (pFUS1-lacZ and pFUS1-Luciferase).

Aequorin is used as a transduction pathway dependent reporter: the Aequorin reporter gene is then expressed only in case of activation of the transduction pathway of interest. For instance, to measure the activity of the pheromone responsive pathway, the expression of the reporter gene Aequorin is controlled by a promoter containing some Pheromone Responsive Elements. The FUS1 promoter is the most commonly utilized. In haploid Saccharomyces cerevisiae cells, GPCRs are regulating the mating process. The receptor Ste2 detects the presence of cells of the opposite mating type (through binding of peptide mating pheromones), and activates intracellular heterotrimeric G proteins, thus initiating the mating process. Gpa1 (α subunit) dissociates from the βγ (Ste4-Ste18) complex which activates downstream elements of the pheromone response pathway which includes a well-characterized mitogen-activated protein kinase (MAP kinase) cascade. The activated transcription factor Ste12 can then initiate the transcription of several mating factor-inducible genes such as *FUS1*.

Controlled by a pheromone dependent gene promoter, Aequorin is expressed proportionally to the activation of the yeast pheromone transduction pathway. Aequorin detection signal quantifies the activity of the pathway.

Pheromone response elements are necessary and sufficient for basal and pheromone-induced transcription of the FUS1 gene of Saccharomyces cerevisiae (Hagen *et al*., 1991).

The present invention relates to a modified yeast cell comprising an Aequorin encoding sequence under the control of an promoter of a mating factor-inducible gene, the FUS1 promoter or a part thereof, e.g. 4PRE. In a special embodiment of the invention the Aequorin encoding sequence is SEQ ID NO. 1. In another special embodiment of the invention to modified yeast cell expresses another heterologous protein, preferably a heterologous protein to be investigated. Examples for such other heterologous proteins are cell surface proteins, e.g. G protein coupled receptors or kinases.

Yeast cells which can be modified are for example *Saccharomyces cerevisiae, Schizosaccharomyces pombe* and *candida albicans.*

The present invention also relates to the use of the modified yeast cells, e.g. for screening of compounds.

The present invention relates to a method for identifying compounds that modulate heterologous cell surface protein-mediated Aequorin expression, wherein
(a) a modified yeast cell comprising an Aequorin encoding sequence under the control of the promoter of a mating factor-inducible gene
(b) the modified yeast cell is incubated with a compound and
(c) the amount of aequorin expression is determined.

Our observations show that Aequorin is superior to β-Galactosidase in a transduction pathway dependent reporter assay.
- Figure 1:: Aequorin - open reading frame (SEQ ID NO. 1)

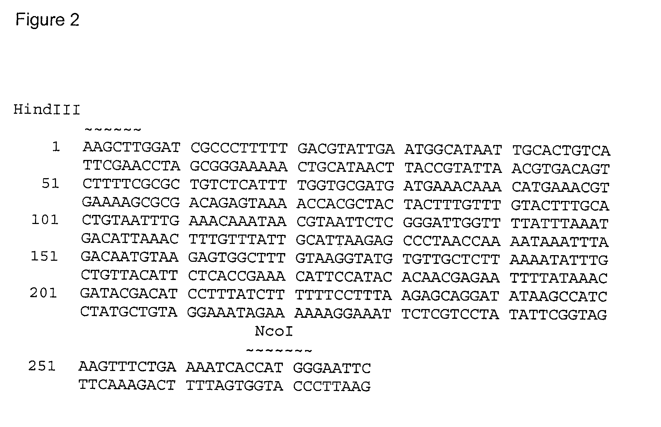

- Figure 2:: 4PRE sequence (SEQ ID NO. 2)
- Figure 3:: Restriction map of p78 4PRE-Aeq
- Figure 4:: Restriction map of p78 4PRE-lacZ
- Figure 5:: Results of pathway activity reporter/ Aequorin activity
- Figure 6:: Results of pathway activity reporter / β-Galactosidase activity
- Figure 7:: Ca²⁺-dependent generation of luminescence by the aequorin complex, which contains apoaequorin (APO) and coelenterazine (Ohmiya and Hirano, 1996)).

### Examples:

### Example 1: Materials and Methods

As a yeast strain W303 MAT a, far::hisG, sst2::URA3^{FOA}, fus1::HIS3 was used.

The yeast strain was transformed with the different plasmids according to the Lithium acetate method (Ito *et al*., 1983)).

### Example 2: Construction of the Aequorin expression vectors

The full length cDNA Aequorin gene was amplified by PCR using the chimerical mitochondrial Aequorin mtAEQ (where the truncated N-terminus is fused to the human cytochrome C targeting signal (Rizzuto *et al*., 1992)) as a template. The 5' PCR primer contained the 50 first nucleotides of aequorin wild type sequence and an EcoRI (noted in bold type below) cloning site. The 3' primer did not contain any cloning site.

5' AQWT: 5'-CCG GAA TTC CGG ATG ACA AGC AAA CAA TAC TCA GTC AAG
CTT ACA TCA GAC TTC GAC AAC CC (SEQ ID NO. 3).

3' AQWT: 5'-GGG CCT TAG GGG ACA GCT CCA CCG TAG AGC (SEQ ID NO. 4).

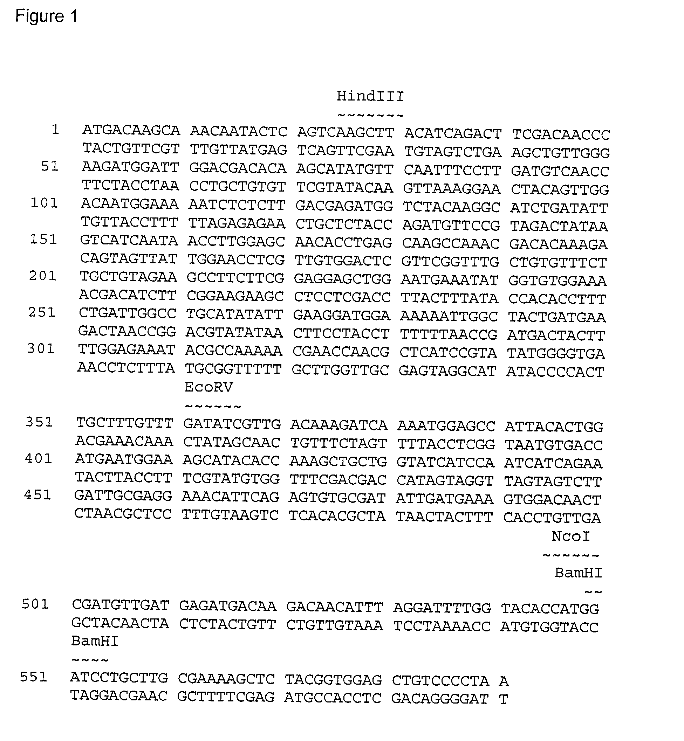

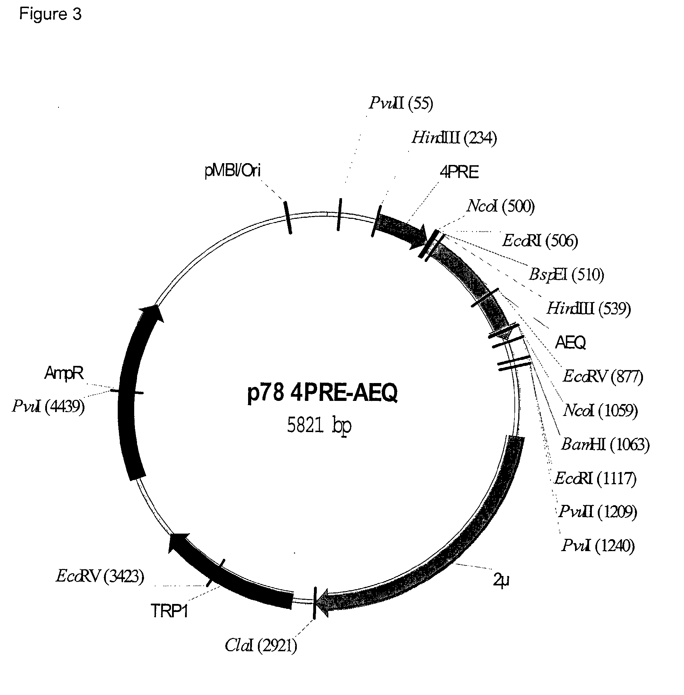

The full-length wild type Aequorin coding sequence (Figure 1) was then cloned in the pheromone pathway activity dependent expression vector p78-4PRE (TRP1, 2µ) (FIG 3).

The minimal and sufficient portion of the FUS1 promoter (Hagen *et al*., 1991) that we called 4PRE is consisting of the last 261 bp of the promoter region, upstream of the FUS1 open reading frame. This promoter was amplified from wild-type yeast genomic DNA with the two following primers:

261 sens Fus: 5'-CTA AAG CTT GGA TCG CCC TTT TTG ACG TAT TGA
(SEQ ID NO. 5)

1Fus rev: 5'-A GA ATT CCC ATG GTG ATT TTC AGA AAC TTG ATG GCT T
(SEQ ID NO. 6).

### Example 3: Construction of the β-Galactosidase expression vectors

To allow the comparison, LacZ was sub-cloned in the same way than aequorin, into the same expression vector and without any fusion with the 5' sequence of a endogenous gene (as is was often done to increase the expression level (King *et al*., 1990))).

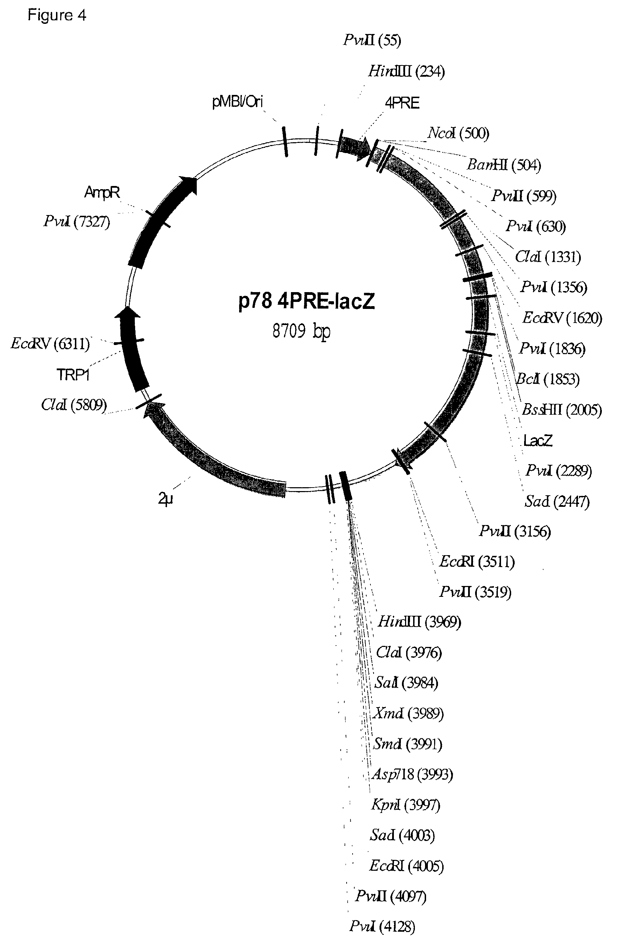

The full-length *Escherichia coli* β-Galactosidase gene sequence was cloned in the pheromone pathway activity dependent expression vector p78-4PRE (TRP1, 2µ) (FIG 4).

### Example 4: Aequorin detection

The cells are distributed and/or grown in a white 96-well plate, in a volume of 100µl.

30 minutes before the measurement time point, 10µl of a 5µM coelenterazine (Molecular Probes) solution is distributed in each well (to obtain a 0.5µM final concentration) to load the cells.

The plate is then incubated at 30°C for the last 30 minutes.

Aequorin detection is made in a luminometer with injecting system (Luminoskan, Labsystems). For each well, immediately after injection of a 10mM CaCl2 solution (1M CaCl2 diluted in lysis buffer Y-PER from Pierce), the luminescent signal is integrated for 15 seconds.

To avoid the intermediate step of loading, it is possible to introduce coelenterazine in the medium at the beginning of the assay: coelenterazine is added at a concentration of 0.5µM.

### Example 5: β-Galactosidase detection

The cells are distributed and/or grown in a white 96-well plate, in a volume of 100µl. At the measurement time point, each well receives 100µl of a β-Galactosidase detection mix (Gal-screen, Tropix).

The plate is incubated for one hour at 28°C.

β-Galactosidase signal is read in a luminometer ; the luminescent signal is integrated for 0.5 seconds.

### Example 6: Classical reporter of a pathway activity

In this assay, the expression of the two reporter genes depends on the activity of the pheromone mating pathway (Leberer *et al*., 1997). The minimal promoter 4PRE amplified from the FUS1 promoter region (Hagen *et al*., 1991) is activated only in case of a mating signal. This signal is elicited by stimulation of the pheromone receptor Ste2 by its ligand α-factor.

Three colonies of the yeast strain transformed with either p78 4PRE-AEQ or p78 4PRE-LacZ plasmids were grown in the appropiate medium (SC Glucose -Trp) to stationary phase and then diluted in the same medium (with 0.5µM coelenterazin for the aequorin strain) containing 0; 10⁻⁷; 10⁻⁹; 10⁻¹¹M α-factor (Sigma). The plates were then shaken at 30°C, 700 r.p.m., for 3, 6 and 24 hours.

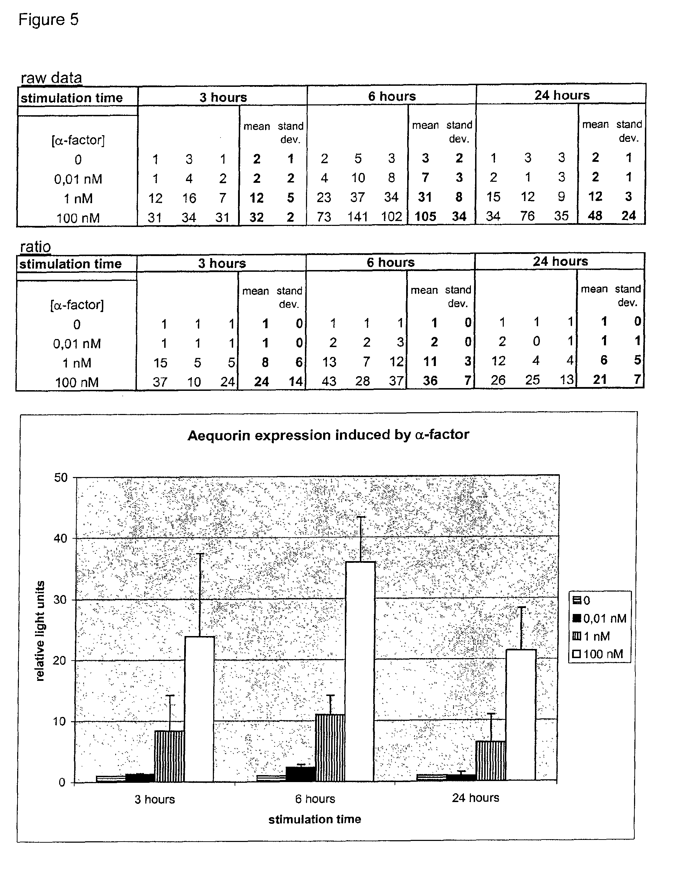

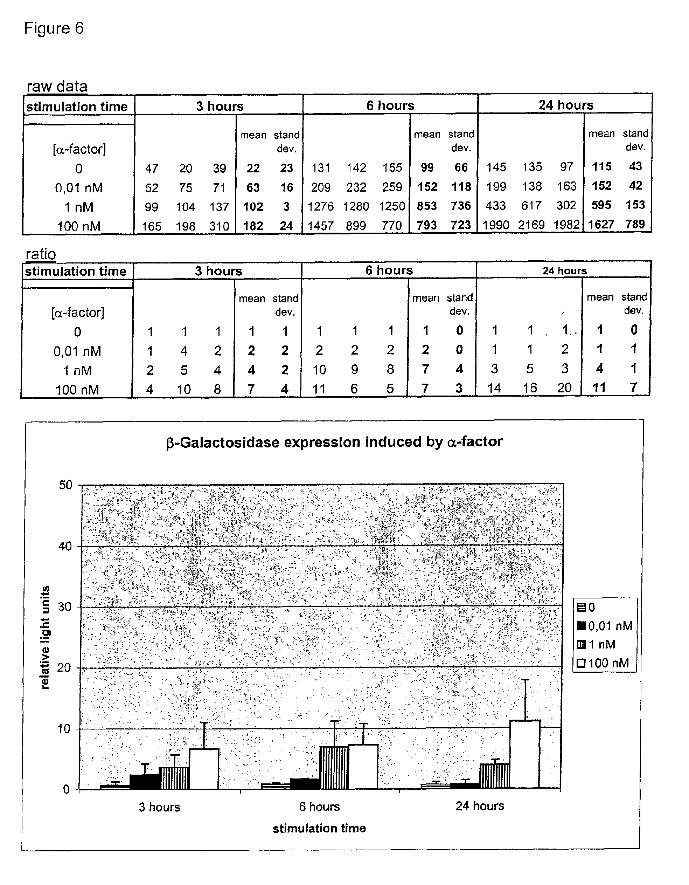

To allow the comparison between the two reporters and the different stimulation times, the measured detection numbers of Aequorin (FIG 5) and β-Galactosidase (FIG 6) are expressed as a ratio: stimulated / non stimulated.

After 3 hours of stimulation, the detected ratio is already higher for Aequorin than for β-Galactosidase with 1 nM or 100 nM α-factor (ratio respectively of 8 and 24 with Aequorin, 4 and 7 with β-Galactosidase). After 6 hours of stimulation, the β-Galactosidase ratio stays at the same level (ratio of 7 for both α-factor concentrations) but Aequorin detection shows higher levels (ratio of 11 with 1 nM α-factor and 36 with 100 nM α-factor). After 24 hours stimulation, Aequorin numbers stay higher than β-Galactosidase.

All together, this experiment shows that Aequorin reflects better than β-Galactosidase the stimulation of Ste2 with different concentrations of ligand, among a large time frame.

### Reference List:

Brini M, Pinton P, Pozzan T and Rizzuto R (1999) Targeted Recombinant Aequorins: Tools for Monitoring [Ca²⁺] in the Various Compartments of a Living Cell. *Microsc Res Tech* **46:** pp 380-389.
Detheux M (2000) Orphan Receptors: the Quest for New Drug Targets. *Innovations in Pharmaceutical Technology* **00:** pp 27-34.
Hadcock JR and Pausch M (1999) Ligand screening of G protein-coupled receptors in yeast, in *G Protein-Coupled Receptors* (Haga T and Berstein G eds) pp 49-69, CRC Press LLC, Boca Raton, Florida.
Hagen DC, McCaffrey G and Sprague G F J (1991) Pheromone Response Elements Are Necessary and Sufficient for Basal and Pheromone-Induced Transcription of the FUS1 Gene of Saccharomyces Cerevisiae. *Mol Cell Biol* **11:** pp 2952-2961.
lida H, Yagawa Y and Anraku Y (1990) Essential Role for Induced Ca2+ Influx Followed by [Ca2+]i Rise in Maintaining Viability of Yeast Cells Late in the Mating Pheromone Response Pathway. A Study of [Ca2+]i in Single Saccharomyces Cerevisiae Cells With Imaging of Fura-2. *J Biol Chem* **265:** pp 13391-13399.
Inouye S, Aoyama S, Miyata T, Tsuji F I and Sakaki Y (1989) Overexpression and Purification of the Recombinant Ca²⁺-Binding Protein, Apoaequorin. *J Biochem (Tokyo)* **105:** pp 473-477.
Ito H, Fukuda Y, Murata K and Kimura A (1983) Transformation of Intact Yeast Cells Treated With Alkali Cations. *J Bacteriol* **153:** pp 163-168.
Johnson FH and Shimomura O (1978) Bioluminescence and Chemiluminescence: Introduction to the Bioluminescence of Medusae, With Special Reference to the Photoprotein Aequorin. *Methods Enzymol* **57:** pp 1-653.
King K, Dohlman H G, Thorner J, Caron M G and Lefkowitz R J (1990) Control of Yeast Mating Signal Transduction by a Mammalian B₂-Adrenergic Receptor and Gs Alpha Subunit [Published Erratum Appears in Science 1991 Jan 11;251(4990):144]. *Science* **250:** pp 121-123.
Leberer E, Thomas D Y and Whiteway M (1997) Pheromone Signalling and Polarized Morphogenesis in Yeast. *Curr Opin Genet Dev* **7:** pp 59-66.
Miller AL, Karplus E and Jaffe L F (1994) Imaging [Ca²⁺]i With Aequorin Using a Photon Imaging Detector. *Methods Cell Biol* **40:** pp 305-338.
Nakajima-Shimada J, lida H, Tsuji F I and Anraku Y (1991a) Galactose-Dependent Expression of the Recombinant Ca2(+)-Binding Photoprotein Aequorin in Yeast. *Biochem Biophys Res Commun* **174:** pp 115-122.
Nakajima-Shimada J, lida H, Tsuji F I and Anraku Y (1991b) Monitoring of Intracellular Calcium in Saccharomyces Cerevisiae With an Apoaequorin CDNA Expression System. *Proc Natl Acad Sci U S A* **88:** pp 6878-6882.
Ohmiya Y and Hirano T (1996) Shining the Light: the Mechanism of the Bioluminescence Reaction of Calcium-Binding Photoproteins. *Chem Biol* **3:** pp 337-347.
Rizzuto R, Simpson A W, Brini M and Pozzan T (1992) Rapid Changes of Mitochondrial Ca2+ Revealed by Specifically Targeted Recombinant Aequorin [Published Erratum Appears in Nature 1992 Dec 24- 31;360(6406):768]. *Nature* **358:** pp 325-327.
Sheu YA, Kricka L J and Pritchett D B (1993) Measurement of Intracellular Calcium Using Bioluminescent Aequorin Expressed in Human Cells. *Anal Biochem* **209:** pp 343-347.
Shimomura O and Johnson F H (1978) Peroxidized Coelenterazine, the Active Group in the Photoprotein Aequorin. *Proc Natl Acad Sci U S A* **75:** pp 2611-2615.
Stables J, Mattheakis L C, Chang R and Rees S (2000) Recombinant Aequorin As Reporter of Changes in Intracellular Calcium in Mammalian Cells. *Methods Enzymol* **327:** pp 456-471.
Thomas AP and Delaville F (1991) The Use of Fluorescent Indicators for measurements of cytosolic-free calcium concentration in cell populations and single cells., in *Cellular Calcium: A Practical Approach* (McCormack JG and Cobbold PH eds) pp 1-54.

## Claims

1. Modified yeast cell comprising an aequorin encoding sequence expressed under control of the promoter of a mating factor-inducible gene.

2. Method for identifying compounds that modulate heterologous cell surface protein-mediated aequorin expression, wherein
(a) a modified yeast cell comprising an aequorin encoding sequence under the control of the promoter of a mating factor-inducible gene
(b) the modified yeast cell is incubated with a compound and
(c) the amount of aequorin expression is determined.
